# EUROPEAN PATENT APPLICATION

(11) **EP 4 141 121 A1**
(43) Date of publication of application: **01.03.2023**
(21) Application number: 21793579.0
(22) Date of filing: 22.04.2021
(51) Int. Cl.: C12N 15/82, C07K 14/005

(54) **INFLUENZA VIRUS SURFACE PROTEIN-DERIVED RECOMBINANT HEMAGGLUTININ PROTEIN FORMING TRIMER, AND USE THEREOF**

(30) Priority: 22.04.2020 KR 20200048979; 08.12.2020 KR 20200170828
(71) Applicant: POSTECH Research and Business Development Foundation, Gyeongsangbuk-do 37673 (KR); Bioapplications Inc., Gyeongsangbuk-do 37668 (KR); Industry-Academic Cooperation Foundation Gyeongsang National University, Jinju-si, Gyeongsangnam-do 52828 (KR); Konkuk University Industrial Cooperation Corp., Seoul 05029 (KR)
(72) Inventor: HWANG, In Hwan, Pohang-si Gyeongsangbuk-do 37673 (KR); LEE, Jun, Ho, Pohang-si Gyeongsangbuk-do 37666 (KR); DIAO, Haiping, Pohang-si Gyeongsangbuk-do 37673 (KR); SONG, Shijian, Pohang-si Gyeongsangbuk-do 37673 (KR); KIM, Woe, Yeon, Jinju-si Gyeongsangnam-do 52849 (KR); KIM, Min, Gab, Jinju-si Gyeongsangnam-do 52849 (KR); RYU, Gyeong, Ryul, Jinju-si Gyeongsangnam-do 52855 (KR); SHIN, Gyeong, Im, Jinju-si Gyeongsangnam-do 52827 (KR); SONG, Chang, Seon, Seoul 05698 (KR); LEE, Ji, Ho, Seoul 05039 (KR); KIM, Deok Hwan, Icheon-si Gyeonggi-do 17330 (KR)
(74) Representative: Dehns
(86) International application number: PCT/KR2021/005119
(87) International publication number: WO 2021/215855

(57) **Abstract**

The present invention relates to an influenza virus surface protein-derived recombinant hemagglutinin (HA) protein forming a trimer and use thereof, and more specifically to a recombinant vector for producing an influenza virus surface protein-derived HA protein forming a trimer, a transformant transformed by the recombinant vector, a method for producing an influenza virus surface protein-derived HA protein forming a trimer using the recombinant vector, an influenza virus surface protein-derived HA protein produced by the method, and the use thereof in preventing, ameliorating or treating influenza virus-infected disease.

## Description

### [Technical Field]

The present invention relates to an influenza virus surface protein-derived recombinant hemagglutinin (HA) protein forming a trimer and use thereof, and more specifically to a recombinant vector for producing an influenza virus surface protein-derived HA protein forming a trimer, a transformant transformed by the recombinant vector, a method for producing an influenza virus surface protein-derived HA protein forming a trimer using the recombinant vector, an influenza virus surface protein-derived HA protein produced by the method, and the use thereof in preventing, ameliorating or treating influenza virus-infected disease.

### [Background Art]

Recently, the possibility of low-cost production of recombinant proteins in plants has been proposed, and various attempts have been made therefor. In particular, studies are being conducted to confirm the production potential of various medical proteins and the like. There may be various advantages in the production of recombinant proteins in plants, one of which is that there are almost no toxins such as endotoxins which are present in microorganisms such as *E*. *coli,* and there are no pathogens that can infect the human body. In addition, since it is known that there are no harmful proteins such as prions, it is possible to produce recombinant proteins that are safer than animal cells or microorganisms. In terms of manufacturing cost, it is much cheaper than animal cells, and it is more economical than microorganisms such as *E. coli* in large-scale production depending on the method of cultivating plants. In order to realize this possibility, it is necessary to develop several essential technologies. Among them, the first and most important technology is the development of an expression vector capable of inducing high gene expression in plants. In plants, gene expression can be induced through various methods. Various methods are possible, such as a method of integrating a recombinant gene into the genome of a plant, a method of fusing with the genome of a chloroplast, and a method of transiently expressing a gene using *Agrobacterium.* The method of fusing a recombinant gene with the nuclear genome or the chloroplast genome basically produces a protein in a plant through the process of securing a transformant. On the other hand, when *Agrobacterium* penetrates plant tissue to induce transient expression of a gene to produce protein, the production process of the transformant is not included, and thus, the protein production period is short, and in general, there is an advantage that the level of protein production is significantly higher than that of protein production through transformants. In addition, since the expression suppression mechanism of other genes possessed by plants can be suppressed by co-infiltration of gene silencing inhibitory factors, it is possible to induce a higher protein expression level. However, whenever transient expression is desired, there is a disadvantage that the *Agrobacterium* culture in which the binary vector containing the target gene is introduced and the *Agrobacterium* culture in which the binary vector expressing the p38 gene silencing suppressor is introduced must be separately prepared and mixed at an appropriate ratio to perform the co-infiltration process. In particular, in the case of culturing two types of *Agrobacterium,* there are limitations in terms of time and economic feasibility.

Influenza virus is an RNA virus belonging to the family *Orthomyxoviridae,* which causes inflammation in the respiratory tract, and it is a highly contagious virus that can be transmitted directly into the air through coughing and saliva of an infected person, or indirectly transmitted to others by contact with an influenza patient. The incubation period is about 24 to 30 hours, and the serotypes of the virus are divided into type A, type B and type C. Among them, type B and type C infection is confirmed only in humans, and type A infection has been confirmed in humans, horses, pigs, other mammals and various kinds of poultry and wild birds. Therefore, it is necessary to develop a vaccine for preventing infection of the influenza virus having such a strong infectious power.

Recombinant protein antigens as vaccines have excellent safety in production and utilization, but have low immunogenicity and generally high production costs compared to live virus-based vaccines. Therefore, in order to increase the efficacy as a vaccine using this highly safe recombinant protein, it is essential to develop a delivery technology for a recombinant protein vaccine capable of inducing various immune responses and inducing high immune responses. In addition, if it can deliver not only one type of antigen but also multiple types of antigens at the same time, it may be a more effective vaccine. In fact, the recent trend is to develop several types of antigens as a single injection. The most effective method to increase the immunogenicity of an antigen is to use a strong adjuvant. If the efficacy of the adjuvant is high, it is possible to effectively induce an immune response even with a small amount of antigen, thereby lowering the price of the vaccine, and thus, the development of potent adjuvants is critical to the development of protein-based vaccines. In addition, since different types of adjuvants can induce different immune responses, it is very important to use an appropriate adjuvant depending on the type of antigen. Currently, injection adjuvants such as aluminum hydroxide have been developed and used in the human body, and cholera toxin B subunit (CTB) and the like are used for oral vaccines. For animals such as livestock, more types of adjuvants have been developed and used. For experiments, Freund's complete adjuvant is widely used for mice. However, it is not yet clear how they enhance the immunogenicity of antigens in humans, livestock and laboratory animals.

Various types of adjuvants are being developed, and since vaccine delivery methods are also diverse, different types of adjuvants are required according to these various delivery methods. Recently, many studies have been conducted using bacteria as an oral vaccine delivery system and adjuvant. In particular, *Lactococcus* is considered to be safe for the human body as bacteria that has secured a 'generally recognized as safe (GRAS) status' by the US Food and Drug Administration (FDA), and it is being developed as an oral adjuvant and antigen delivery agent. Since bacteria themselves are very antigenic, it has been reported that they show a very high immune response to the antigens delivered by the bacteria.

The full-length protein of hemagglutinin (HA) is a membrane-bound form having a transmembrane domain, and it is difficult to produce at a high level in cells. On the other hand, if only the ectodomain is expressed except for the transmembrane domain of HA, it can be made in a soluble form in the cell and produced with high efficiency. However, when only the ectodomain of HA is produced in a soluble form, the trimeric form of the original full-length HA when it is present on the surface of the influenza virus is not well made. In order to use for vaccine purposes, it was attempted to develop a technique for inducing the formation of trimers when expressing and producing recombinant proteins of the ectodomain of HA in plants. In addition, HA ectodomain recombinant proteins have been devised to have the ability to bind the protein produced in this way to peptidoglycan and to deliver antigens in various ways by binding genes capable of binding to the surface of *Lactococcus* or chitosan particles. A binary vector was constructed to allow high expression of the recombinant gene of HA produced in this way in plants. The treatment effect was confirmed as a result of treating the influenza virus-infected mice with the HA recombinant protein highly expressed in the plant, and furthermore, *Lactococcus* having CTB, which is known to significantly increase the HA recombinant protein antigen and immune response, in a GRAS status were heated and treated with trichloroacetic acid to remove soluble proteins and nucleic acids of the bacteria, and then, as a result of coating *Lactococcus* dead cells and treating influenza virus-infected mice with an injection vaccine, it was confirmed that the therapeutic effect was increased. Furthermore, as a result of treating influenza virus-infected chickens with the HA trimer of water-soluble H5N6 or the HA trimer of H5N6 coated on the surface of *Lactococcus* dead cells as an injection vaccine, a high immune effect was confirmed. CTB, the HA trimer of soluble H5N6 and the HA trimer of soluble H9N2 were coated on *Lactococcus* dead cells, respectively, and then mixed to prepare an immune composition and analyzed for hemagglutination, and as a result, it was confirmed that hemagglutination increased in the group containing CTB, and that CTB increased immunogenicity. After preparing a vaccine composition by mixing *Lactococcus* coated with the HA trimer of H5N6 and *Lactococcus* coated with the HA trimer of H9N2 at a ratio of 1:1, as a result of immunizing mice using the same, it was confirmed that the immunogenicity increased. After mixing the HA trimer of H5N6 and the HA trimer of H9N2 at a ratio of 1:1, it was coated on *Lactococcus* and was prepared as a vaccine composition and immunized into mice, and as a result, it was confirmed that the immunogenicity was increased.

### [Disclosure]

### [Technical Problem]

The present invention has been devised to solve the above problems, and an object of the present invention is to provide a recombinant vector for producing an influenza virus-derived recombinant hemagglutinin (HA) protein forming a trimer, comprising (i) a gene encoding a protein lacking a transmembrane protein portion in influenza virus-derived hemagglutinin (HA); and (ii) a gene encoding a protein of a trimeric motif region of Coronin 1.

Another object of the present invention is to provide a recombinant vector for producing an influenza virus-derived recombinant HA protein forming a trimer, in which a gene encoding a protein of the LysM domain is further inserted into the aforementioned recombinant vector.

Another object of the present invention is to provide a method for producing an influenza virus-derived recombinant hemagglutinin (HA) protein forming a trimer in a plant, comprising the steps of:
(a) constructing the aforementioned recombinant vector;
(b) introducing the recombinant vector into a cell to prepare a transformant;
(c) culturing the transformant;
(d) infiltrating a plant with a culture product in which the transformant is cultured; and
(e) pulverizing the plant to obtain an influenza virus-derived recombinant hemagglutinin (HA) protein forming a trimer.

Another object of the present invention is to provide an influenza virus-derived recombinant HA protein forming a trimer, which is produced by the aforementioned method.

Still another object of the present invention is to provide a vaccine composition with increased immunogenicity, for preventing or treating influenza virus-infected disease, comprising the aforementioned influenza virus-derived recombinant hemagglutinin (HA) protein forming a trimer.

Another object of the present invention is to provide a vaccine composition with increased immunogenicity, for preventing or treating influenza virus-infected disease, comprising bacteria or chitosan coated with the aforementioned influenza virus-derived recombinant HA protein forming a trimer.

Still another object of the present invention is to provide a vaccine composition with increased immunogenicity, for preventing or treating influenza virus-infected disease, in which the aforementioned vaccine composition further comprises a cholera toxin B subunit.

Another object of the present invention is to provide a vaccine composition with increased immunogenicity, for preventing or treating virus-infected diseases caused by influenza viruses having different genotypes, comprising two or more different types of influenza virus-derived recombinant HA proteins forming the aforementioned trimer.

Still another object of the present invention is to provide a method for preventing or treating influenza virus-infected disease, comprising administering the various types of vaccine compositions described above to a subject in need thereof.

### [Technical Solution]

In order to solve the above problems, the present invention provides a recombinant vector for producing an influenza virus-derived recombinant hemagglutinin (HA) protein forming a trimer, including (i) a gene encoding a protein lacking a transmembrane protein portion in influenza virus-derived hemagglutinin (HA); and (ii) a gene encoding a protein of a trimeric motif region of Coronin 1.

According to a preferred exemplary embodiment of the present invention, the influenza virus may be any one or more selected from the group consisting of influenza A viruses H5N6, H7N9 and H9N2.

According to another preferred exemplary embodiment of the present invention, the protein lacking a transmembrane protein portion in the influenza virus-derived HA may include the amino acid sequence of SEQ ID NO: 2 or the amino acid sequence of SEQ ID NO: 18.

According to still another preferred exemplary embodiment of the present invention, the gene encoding a protein lacking a transmembrane protein portion in the influenza virus-derived HA may include the nucleotide sequence of SEQ ID NO: 1 or the nucleotide sequence of SEQ ID NO: 17.

According to another preferred exemplary embodiment of the present invention, the protein of a trimeric motif region of Coronin 1 may include the amino acid sequence of SEQ ID NO: 4.

According to still another preferred exemplary embodiment of the present invention, the gene encoding the protein of a trimeric motif region of Coronin 1 may include the nucleotide sequence of SEQ ID NO: 3.

According to another preferred exemplary embodiment of the present invention, a gene encoding a protein of the LysM domain may be further inserted into the aforementioned recombinant vector.

According to still another preferred exemplary embodiment of the present invention, the protein of the LysM domain may include the amino acid sequence of SEQ ID NO: 14.

According to another preferred exemplary embodiment of the present invention, the gene encoding the protein of the LysM domain may include the nucleotide sequence of SEQ ID NO: 13.

According to still another preferred exemplary embodiment of the present invention, the recombinant vector may further include any one promoter selected from the group consisting of a 35S promoter derived from cauliflower mosaic virus, a 19S RNA promoter derived from cauliflower mosaic virus, a Mac promoter, an actin protein promoter and ubiquitin protein promoter of a plant.

The present invention also provides a transformant which is transformed with the aforementioned recombinant vector.

According to a preferred exemplary embodiment of the present invention, the transformant may be a prokaryote or a eukaryote.

Further, the present invention provides a method for producing an influenza virus-derived recombinant HA protein forming a trimer in a plant, including the following steps, and an influenza virus-derived recombinant HA protein forming a trimer produced therefrom.

Furthermore, the present invention provides a vaccine composition with increased immunogenicity, for preventing or treating influenza virus-infected disease, comprising the aforementioned influenza virus-derived recombinant HA protein forming a trimer.

According to a preferred exemplary embodiment of the present invention, the influenza virus-derived recombinant HA protein forming a trimer may be coated on the surface of bacteria including peptidoglycan in the cell wall or chitosan.

According to another preferred exemplary embodiment of the present invention, the bacteria including peptidoglycan in the cell wall may be bacteria that are generally recognized as safe (GRAS).

According to still another preferred exemplary embodiment of the present invention, the vaccine composition may further include a cholera toxin B subunit.

According to another preferred exemplary embodiment of the present invention, the vaccine composition may be an injection form.

In addition, the present invention provides a vaccine composition with increased immunogenicity, for preventing or treating influenza virus-infected diseases caused by influenza viruses having different genotypes, comprising two or more different types of influenza virus-derived recombinant HA proteins that form the above-mentioned trimer.

According to a preferred exemplary embodiment of the present invention, the recombinant HA protein derived from two or more different types of influenza viruses forming the trimer may be an HA protein derived from any one or more influenza viruses selected from the group consisting of H5N6, H7N9 and H9N2.

According to another preferred exemplary embodiment of the present invention, the HA protein derived from two or more different types of influenza viruses forming the trimer may be coated on the surface of bacteria including peptidoglycan in the cell wall or chitosan.

According to still another preferred exemplary embodiment of the present invention, the bacteria including peptidoglycan in the cell wall may be bacteria that are generally recognized as safe (GRAS).

According to another preferred exemplary embodiment of the present invention, the two or more different types of influenza virus-derived recombinant HA proteins forming a trimer may be coated on the surface of bacteria comprising peptidoglycan in the cell wall or chitosan by any one method of i) to iii) below:
i) coating the surface of bacteria comprising peptidoglycan in the cell wall or chitosan, after mixing two or more different types of influenza virus-derived recombinant HA proteins forming a trimer;
ii) coating the surface of bacteria comprising peptidoglycan in the cell wall or chitosan with each of two or more different types of influenza virus-derived recombinant HA proteins forming a trimer, followed by mixing; or
iii) coating the surface of bacteria comprising peptidoglycan in the cell wall or chitosan with two or more different types of influenza virus-derived recombinant HA proteins forming a trimer by the two methods of (i) and (ii) above.

According to still another preferred exemplary embodiment of the present invention, the vaccine composition may further comprise a cholera toxin B subunit.

Furthermore, the present invention provides a method for preventing or treating influenza virus-infected disease, comprising administering the aforementioned various types of vaccine compositions to a subject in need thereof.

### [Advantageous Effects]

The influenza virus-derived recombinant HA protein forming a trimer according to the present invention includes a protein of the ectodomain region lacking a transmembrane protein in HA derived from highly pathogenic influenza A virus H5N6, a trimeric motif of Coronin A in mice and the LysM domain, which is the cell wall binding domain of a LysM peptidoglycan-binding domain-containing protein of *Lactococcus lactis,* and it can be prepared in large quantities in plants, increases immunity by forming a trimer, and thus, the antigen can be effectively delivered by binding to or coated with bacteria such as *Lactococcus* or chitosan particles. As a result of treating mice infected with influenza virus with the HA recombinant protein highly expressed in the plant, a therapeutic effect was shown, and in addition, the HA recombinant protein antigen and the cholera toxin B subunit, which is known to significantly increase the immune response, were coated on dead cells prepared by heating *Lactococcus* having a GRAS status and treating with trichloroacetic acid to remove the bacteria's water-soluble proteins and nucleic acids, and as a result of treating with influenza virus-infected mice as an injection vaccine, it showed an excellent therapeutic effect. Furthermore, as a result of treating influenza virus-infected chickens with an injection vaccine by coating the HA trimer of soluble H5N6, the HA trimer of soluble H9N2, or these on the surface of the *Lactococcus* dead cells according to the present invention, it showed a high immune effect. As a result of hemagglutination analysis using an immune composition prepared by coating CTB (cholera toxin B subunit), the HA trimer of water-soluble H5N6 and the HA trimer of water-soluble H9N2 on *Lactococcus* dead cells, respectively and mixing the same, it was confirmed that CTB had the effect of enhancing immunogenicity by increasing hemagglutination in the group including CTB. As a result of immunizing mice with a vaccine composition prepared by mixing the *Lactococcus* dead cells coated with the HA trimer of H5N6 and the *Lactococcus* dead cells coated with the HA trimer of H9N2 at a ratio of 1: 1, it showed the effect of increasing immunogenicity. The vaccine composition prepared by mixing the HA trimer of H5N6 and the HA trimer of H9N2 at a ratio of 1:1 and then coating the same on the *Lactococcus* dead cells was immunized in mice, and as a result, it also showed the effect of increasing immunogenicity. Accordingly, by coating several different types of antigens on *Lactococcus* dead cells and mixing the same, or by simultaneously mixing several different types of antigens and then coating the same on *Lactococcus* dead cells, or by using the above two methods together, immunogenicity can be effectively enhanced by the simultaneous delivery of multiple antigens.

### [Description of Drawings]

FIGS. 1a and 1b are mimetic diagrams showing the construction of a recombinant vector used to produce an influenza virus-derived recombinant HA protein forming a trimer according to the present invention.
FIG. 2a shows the results of performing Western blot analysis using an anti-His antibody, after separating the protein expressed in *Nicotiana benthamiana* by SDS/PAGE.
FIG. 2b shows the results of SDS/PAGE separation of the protein expressed in *Nicotinia benthamiana* and then staining with Coomassie brilliant blue.
FIGS. 3a and 3b show the results of confirming that mH5N6 and tH5N6 formed a monomer and a trimer, respectively, by using gel filtration column chromatography, and specifically, FIG. 3a shows the result of fractionation through gel filtration column chromatography, and FIG. 3b shows the result of performing Western blot analysis using an anti-His antibody after developing the fraction corresponding to each peak by SDS/PAGE.
FIGS. 3c and 3d show the results of confirming that mH9N2 and tH9N2 formed a monomer and a trimer, respectively, using gel filtration column chromatography, and specifically, FIG. 3c shows the result of fractionation through gel filtration column chromatography, and FIG. 3d shows the result of performing Western blot analysis using an anti-His antibody after developing the fraction corresponding to each peak by SDS/PAGE.
FIGS. 4a and 4b confirm the trimer effect of mCor1 on the binding of LysM to *Lactococcus lactis,* and specifically, FIG. 4a shows the results of GFP-LysM and GFP-mCor-LysM binding to *Lactococcus,* respectively, and developing the same by SDS/PAGE, analyzing by Western blot analysis, and staining with Coomassie Brilliant Blue, and FIG. 4b shows the results of observing the degree of binding to *Lactococcus* in which GFP-LysM and GFP-mCor-LysM were treated with TCA, respectively, under a fluorescence microscope.
FIG. 5 shows the results of binding the HA monomer (mH5N6) and trimer (tH5N6) of H5N6 prepared in *Nicotiana benthamiana* leaf cells to *Lactococcus* treated with TCA, and then developing the same by SDS/PAGE and staining with Coomassie Brilliant Blue.
FIG. 6a shows a dosing schedule for confirming the immunogenic response of mice to soluble tHA and tHA coated on the *Lactococcus* surface.
FIG. 6b shows the results of measuring the immunogenic response of mice to soluble tHA and tHA coated on the *Lactococcus* surface by ELISA.
FIGS. 7a and 7b show the results of analyzing the degree of inhibition of hemagglutination by soluble tHA and tHA coated on the surface of *Lactococcus.*
FIG. 8a shows the results of an antibody induction experiment using PBS, *Lactococcus* dead cells, the HA trimer of soluble H5N6 and the HA trimer of H5N6 coated on the surface of *Lactococcus* as antigens for chickens.
FIG. 8b shows the results of an antibody induction experiment using PBS, *Lactococcus* dead cells, the HA trimer of soluble H9N2 and the HA trimer of H9N2 coated on the surface of *Lactococcus* as antigens for chickens.
FIG. 9 shows the results of confirming immunogenicity by mixing *Lactococcus* coated with cholera toxin B subunit (CTB), the HA trimer of soluble H5N6, and the HA trimer of soluble H9N2, respectively.
FIG. 10 shows the results of immunizing mice with a vaccine composition prepared by mixing *Lactococcus* coated with the HA trimer of H5N6 and *Lactococcus* coated with the HA trimer of H9N2 at a ratio of 1:1, and then confirming strong immunogenicity against the two types of the antigens.
FIG. 11 shows the result of immunizing mice with a vaccine composition prepared by mixing the HA trimer of H5N6 and the HA trimer of H9N2 at a ratio of 1:1 and coating the same on *Lactococcus,* and then confirming strong immunogenicity against the two types of the antigens.

### [Best Mode]

Hereinafter, the present invention will be described in detail.

As described above, the full-length protein of influenza virus-derived hemagglutinin (HA) is a membrane-bound form having a transmembrane domain, and there is a limitation in that it is difficult to produce at a high level in cells. Accordingly, in order to increase the production level, if only the ectodomain is expressed except for the transmembrane domain of HA and made into a soluble form in cells, there is an advantage that it can be produced with high efficiency. However, when only the ectodomain of HA is prepared in a soluble form, there is a limitation in that the trimeric form of the original full-length HA is not well made when it is present on the surface of the influenza virus. Accordingly, the inventors of the present invention tried to develop a technique for inducing the formation of a trimer when expressing and producing a recombinant protein of the ectodomain of HA in plants for the purpose of use as a vaccine with increased immunogenicity. In addition, in order for the protein thus produced to have the ability to bind to peptidoglycan, it was attempted to develop a recombinant HA ectodomain protein capable of delivering antigens in various ways by binding with genes capable of binding to surfaces such as *Lactococcus* or chitosan particles. Accordingly, by designing HA expressing only the ectodomain excluding the transmembrane domain of influenza virus-derived HA that can be mass-produced, a trimeric motif of Coronin A in mice forming a trimeric structure and a *Lactococcus lactis* LysM peptidoglycan-binding domain-including protein that binds to the surface of *Lactococcus* or chitosan particles to effectively deliver an antigen, and expressing them in one vector, an influenza virus-derived recombinant HA protein forming a trimer and a binary vector capable of expressing the protein in a large amount in plants were constructed.

Accordingly, a first aspect of the present invention relates to a recombinant vector for producing an influenza virus-derived recombinant hemagglutinin (HA) protein forming a trimer, including (i) a gene encoding a protein lacking a transmembrane protein portion in influenza virus-derived hemagglutinin (HA); and (ii) a gene encoding a protein of a trimeric motif region of Coronin 1.

In the recombinant vector of the present invention, the influenza virus may include, without limitation, various types of influenza viruses that infect humans, dogs, pigs, horses, poultry, wild birds, seals and the like, and may include conventionally known influenza virus types A, B and C, *Isavirus* or *Thogotovirus.*

Influenza virus type A is the cause of seasonal flu and pandemic flu epidemics. Wild aquatic algae are natural hosts for a wide variety of influenza A. Occasionally, the virus can spread to other species, causing devastating outbreaks in poultry or human influenza pandemics. Type A virus is the most lethal human pathogen of the three influenza types and causes the most serious disease. Influenza A viruses can be subdivided into different serotypes based on antibody responses to these viruses. The serotypes identified in humans (in order of the number of known human pandemic deaths) are as follows: H1N1 (cause of the 1918 Spanish influenza), H2N2 (the cause of the 1957 Asian influenza), H3N2 (the cause of the 1968 Hong Kong flu), H5N1 (pandemic threat during the 2007-2008 influenza season), H7N7 (potential pandemic threat), H1N2 (endemic disease in humans and pigs), H9N2, H7N2, H7N3 and H10N7.

Influenza virus type B is the cause of seasonal flu and has one type of influenza B virus. Influenza B infects humans almost exclusively and is less common than influenza A. The only other animal known to be susceptible to infection with influenza virus type B is the seal. Because this type of influenza mutates at a rate that is two to three times slower than type A, it is less genetically diverse and has only one influenza B serotype. As a result of this lack of antigenic diversity, some degree of immunity to influenza B is usually acquired at an early age, but influenza B is mutated sufficiently to prevent sustained immunity.

Influenza virus type C infects humans and pigs and can cause serious illness and local infectious diseases, but is less common than other types and appears to usually cause mild illness in children.

In the present invention, the influenza A virus, for example, may be any one selected from the group consisting of H5N6, H7N9 and H9N2, but is not limited thereto.

In a specific exemplary embodiment of the present invention, a recombinant HA protein forming a trimer was prepared using HA proteins derived from influenza A viruses H5N6 and H9N2. Specifically, in order to increase the productivity of HA, except for the transmembrane domain in the HA of H5N6 or H9N2, the amino acid sequence encoding only the ectodomain, that is, the amino acid sequence including the amino acid residues at positions 17 to 531 of the HA of H5N6 (GenBank: AJD09950.1) and the amino acid sequence including the amino acid residues at positions 19 to 524 of the HA of H9N2 (GenBank: AFM47147.1) were selectively used, respectively. For the recombinant vector according to the present invention, in order to prepare a recombinant HA protein forming a trimer, various amino acid sequences of the region consisting of the amino acid residues at positions 17 to 531 of the HA of H5N6 (GenBank: AJD09950.1) can be selectively used, and in the same way, various amino acid sequences of the region consisting of the amino acid residues at positions 19 to 524 of the HA of H9N2 (GenBank: AFM47147.1) can be selectively used.

In the recombinant vector of the present invention, the protein lacking a transmembrane protein portion in the influenza virus-derived hemagglutinin (HA) may include the amino acid sequence of SEQ ID NO: 2 or the amino acid sequence of SEQ ID NO: 18, but is not limited thereto.

The gene encoding the protein lacking a transmembrane protein portion in the influenza virus-derived HA may include the nucleotide sequence of SEQ ID NO: 1 or the nucleotide sequence of SEQ ID NO: 17, and specifically, the gene may include a nucleotide sequence having at least 70%, more preferably, at least 80%, even more preferably, at least 90%, and most preferably, at least 95% sequence homology to the nucleotide sequence of SEQ ID NO: 1 or the nucleotide sequence of SEQ ID NO: 17, respectively.

The "% of sequence homology" for a polynucleotide is determined by comparing two optimally aligned sequences with a comparison region, and a portion of the polynucleotide sequence in the comparison region may include additions or deletions (i.e., gaps) compared to a reference sequence (not including additions or deletions) to the optimal alignment of the two sequences.

In the recombinant vector of the present invention, Coronin 1 above may be mouse-derived Coronin 1 (mCor 1) (GenBank: EDL17419.1), and the protein of its trimeric motif region may include the amino acid sequence of SEQ ID NO: 4. In the recombinant vector of the present invention, Coronin 1 (mCor 1) is linked to the C-terminus of the ectodomain of influenza virus-derived HA such that a trimer may be formed even when only the ectodomain of HA is expressed.

The gene encoding the protein of the trimeric motif region of Coronin 1 (mCor 1) may include the nucleotide sequence of SEQ ID NO: 3, and specifically, the gene may include a nucleotide sequence having at least 70%, more preferably, at least 80%, even more preferably, at least 90%, and most preferably, at least 95% sequence homology to the nucleotide sequence of SEQ ID NO: 3.

The recombinant vector of the present invention may further include a gene encoding a protein of the LysM domain.

In the recombinant vector of the present invention, the protein of the LysM domain may include the amino acid sequence of SEQ ID NO: 14, and the gene encoding the protein of the LysM domain may include the nucleotide sequence of SEQ ID NO: 13, and specifically, the gene may include a nucleotide sequence having at least 70%, more preferably, at least 80%, even more preferably, at least 90%, and most preferably, at least 95% sequence homology to the nucleotide sequence of SEQ ID NO: 13, respectively.

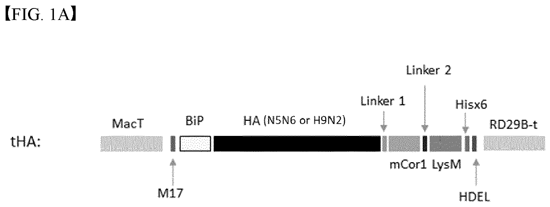

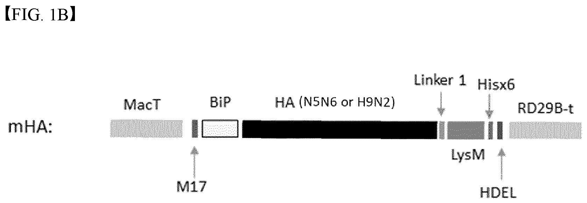

In the influenza virus-derived HA according to the present invention, for the effective delivery of antigens and promoting various immune effects on the recombinant HA protein including the protein lacking a transmembrane protein portion and the protein of the trimeric motif region of Coronin 1 (mCor 1), the 220^{th} to 320^{th} amino acid residues of the LysM domain (GenBank: WP_011834353), which is a cell wall-binding domain of the LysM peptidoglycan-binding domain-including protein of *Lactococcus lactis* capable of binding to bacteria such as *Lactococcus* or chitosan particles, were fused to the C-terminus of the trimeric motif of Coronin 1 (mCor1) using a linker having 6 amino acid residues. Subsequently, a His tag having 6 His residues was fused for isolation and purification of the recombinant protein, and an HDEL motif was fused for ER accumulation to complete the construct tHA (FIG. 1a). As a control group, a construct mHA without the trimer motif of mCor1 was constructed and compared (FIG. 1b).

The recombinant genes of HA thus constructed were introduced into a plant expression vector, pTEX1, to prepare a plant expression vector (pTEX-tHA and pTEX-mHA, respectively). Afterwards, the constructed expression vectors were introduced into *Agrobacterium,* and then infiltrated into plants to induce transient expression. The expressed recombinant HA protein may be separated by a Ni²⁺-NTA affinity column using a His tag or by binding to *Lactococcus* using the LysM domain.

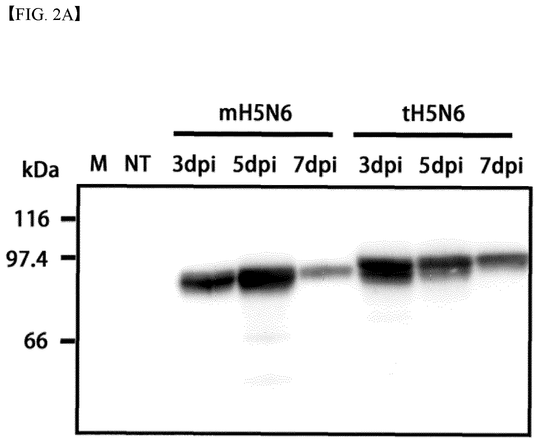

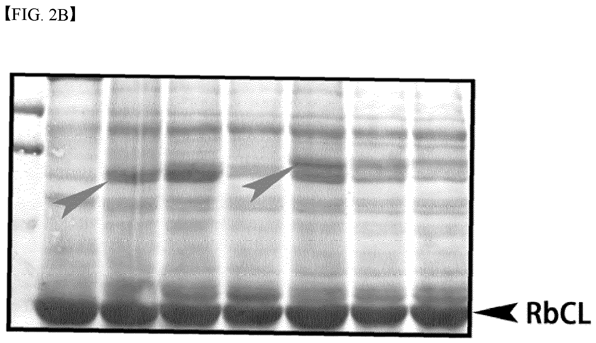

In order to confirm the protein expression in the leaf extract of *Nicotiana benthamiana* into which these genes were introduced, Western blot analysis was performed by using an anti-His antibody. As shown in FIG. 2a, HA-LysM-His-HDEL was identified as a protein at a position of about 80 kDa. This is larger than the computational protein position, and it is thought to be due to N-glycosylation of the HA protein. In addition, it can be seen from FIG. 2b that tHA is slightly larger than mHA.

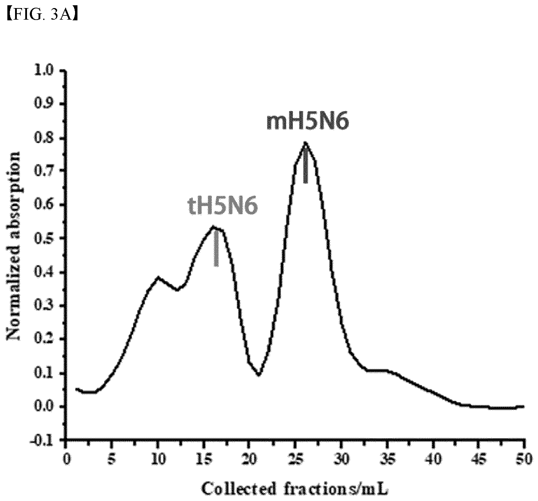

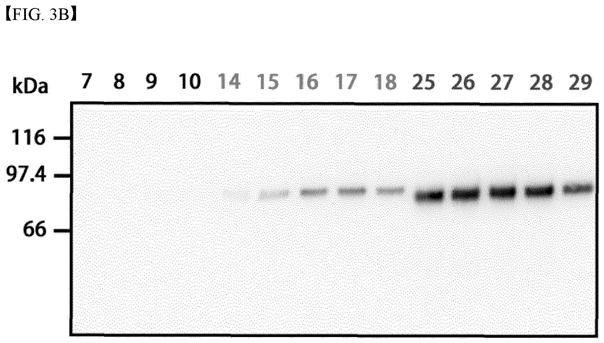

Next, in order to confirm the formation of a trimer of the protein, mHA in a monomeric form and tHA in a trimeric form were mixed and gel filtration was performed. As a result, as confirmed in FIG. 3a, two peaks appeared, and a Western blot was performed on the fraction corresponding to each of these peaks using an anti-His antibody, and as shown in FIG. 3b, the protein of the peak corresponding to the trimer was identified as tHA, and the peak corresponding to the monomer was identified as the mHA protein.

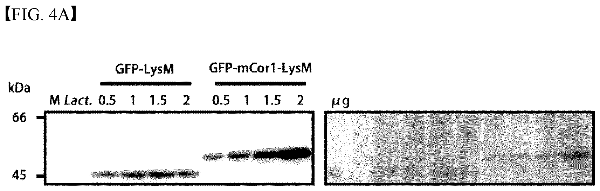

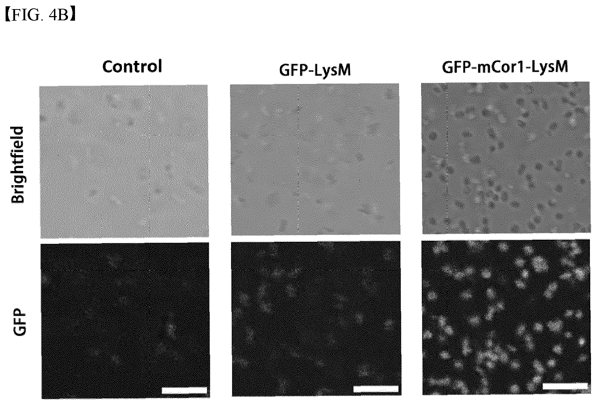

In a specific exemplary embodiment of the present invention, in order to confirm the binding of LysM of the recombinant protein to *Lactococcus,* by using GFP as a control protein, a His-tagged GFP-mCor1-LysM construct and a His-tagged GFP-LysM construct were prepared, and these were introduced into pRSET-A, which is an expression vector of *E. coli,* to construct expression vectors capable of expression in *E. coli.* The proteins GFP-mCor1-LysM and GFP-LysM were expressed in *E. coli,* isolated and purified, and the degree of binding to *Lactococcus* was observed under a fluorescence microscope. As a result, as shown in FIG. 4, it was confirmed that GFP-mCor1-LysM had higher GFP expression than GFP-LysM.

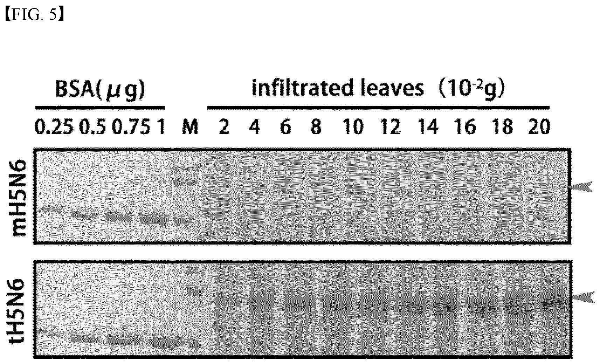

In another specific exemplary embodiment of the present invention, in the *Lactococcus* surface binding of HA by LysM, the trimerization effect by mCor1 was determined. To this end, a total extract was obtained by grinding the leaf tissue of *Nicotiana benthamiana* expressing mHA (mH5N6) in the monomeric form and tHA (tH5N6) in the trimeric form, and it was mixed with *Lactococcus* treated with TCAto induce binding, and after *Lactococcus* was pelleted and recovered therefrom, it developed by SDS/PAGE and stained with Coomassie Brilliant Blue. As a result, as confirmed in FIG. 5, tHA (tH5N6) formed a trimeric HA, whereas mHA (mH5N6) did not form a trimeric HA, and the amount of tHA (tH5N6) that formed the trimeric structure and bound to *Lactococcus* increased as the amount of HA increased, whereas mHA hardly bound to *Lactococcus.*

The recombinant vector of the present invention may further include any one promoter selected from the group consisting of a 35S promoter derived from cauliflower mosaic virus, a 19S RNA promoter derived from cauliflower mosaic virus, a Mac promoter, an actin protein promoter and ubiquitin protein promoter of a plant, and preferably, it may include a Mac promoter, and more preferably, a MacT promoter, but is not limited thereto.

The MacT promoter may be a promoter in which A, which is the 3' terminal base of the Mac promoter nucleotide sequence, is substituted with T, and the MacT promoter may include the nucleotide sequence of SEQ ID NO: 15, and specifically, the gene may include a nucleotide sequence having at least 70%, more preferably, at least 80%, even more preferably, at least 90%, and most preferably, at least 95% sequence homology to the nucleotide sequence of SEQ ID NO: 15.

The recombinant vector of the present invention may further include an RD29B-t termination site, and the RD29B-t termination site gene may include the nucleotide sequence of SEQ ID NO: 16, and specifically, the gene may include a nucleotide sequence having at least 70%, more preferably, at least 80%, even more preferably, at least 90%, and most preferably, at least 95% sequence homology to the nucleotide sequence of SEQ ID NO: 16.

In the recombinant vector of the present invention, by inserting the signal sequence of BiP (chaperone binding protein) and HDEL, which is an ER retention signal at the N-terminus and C-terminus of the gene encoding the recombinant protein, respectively, it is possible to have the effect of inducing accumulation in the endoplasmic reticulum (ER) at high concentrations. Accordingly, the recombinant vector of the present invention may further include a gene encoding a BiP and/or a gene encoding a HDEL (His-Asp-Glu-Leu) peptide, wherein the BiP-encoding gene may include a nucleotide sequence of SEQ ID NO: 9, and HDEL (His-Asp-Glu-Leu) may include the nucleotide sequence of SEQ ID NO: 10.

As used herein, the term "recombinant" refers to a cell in which the cell replicates a heterologous nucleic acid, expresses the nucleic acid, or expresses a peptide, a heterologous peptide, or a protein encoded by the heterologous nucleic acid. Recombinant cells can express genes or gene segments that are not found in the native form of the cells in either the sense or antisense form. Recombinant cells can also express genes found in cells in a natural state, but the genes are modified and reintroduced into cells by artificial means.

The term "recombinant expression vector" means a bacterial plasmid, phage, yeast plasmid, plant cell virus, mammalian cell virus or other vector. In general, any plasmid and vector may be used as long as it can be replicated and stabilized in the host. An important characteristic of the expression vector is that it has an origin of replication, a promoter, a marker gene and a translation control element. The recombinant expression vector and the expression vector including appropriate transcriptional/translational control signals may be constructed by methods well known to those skilled in the art. The methods include *in vitro* recombinant DNA technology, DNA synthesis technology and *in vivo* recombination technology.

A preferred example of the recombinant vector of the present invention is a Ti-plasmid vector capable of transferring a part of itself, the so-called T-region, into a plant cell when present in a suitable host. Another type of Ti-plasmid vector is currently being used to transfer hybrid DNA sequences into plant cells, or protoplasts from which new plants can be produced that properly insert the hybrid DNA into the genome of the plant. A particularly preferred form of the Ti-plasmid vector is the so-called binary vector as claimed in European Patent No. EP 0 120 516 B1 and US Patent No. 4,940,838. Other suitable vectors that can be used to introduce into a plant host a construct encoding the influenza virus-derived recombinant HA protein forming a trimer designed in the present invention may be selected from viral vectors such as those derived from double-stranded plant viruses (e.g., CaMV) and single-stranded virus, geminiviruses and the like, and for example, incomplete plant viral vectors. The use of such vectors can be advantageous, especially when it is difficult to adequately transform a plant host.

A second aspect of the present invention relates to a transformant which is transformed with the aforementioned recombinant vector.

The transformant of the present invention may be a prokaryote or a eukaryote, and for example, yeast (*Saccharomyce cerevisiae*)*, E. coli,* fungi, insect cells, human cells (e.g., CHO (Chinese hamster ovary) cell line, W138, BHK, COS-7, 293, HepG2, 3T3, RIN and MDCK cell lines) and plant cells may be used, and preferably, it may be *Agrobacterium.* In the case of insect cells and human cells, the gene encoding a recombinant HA protein forming a trimer may be expressed using an expression vector required for the expression of each type of cells.

The method of delivering the recombinant vector of the present invention into a host cell may be carried out by the CaCh method, Hanahan method, electroporation method and the like, when the host cell is a prokaryotic cell. In addition, when the host cell is a eukaryotic cell, the vector may be injected into the host cell by microinjection, calcium phosphate precipitation, electroporation, liposome-mediated transfection, DEAE-dextran treatment, gene bombardment and the like.

A third aspect of the present invention relates to a method for producing an influenza virus-derived recombinant HA protein forming a trimer in a plant, including the steps of:
(a) constructing the aforementioned recombinant vector;
(b) introducing the recombinant vector into a cell to prepare a transformant;
(c) culturing the transformant;
(d) infiltrating a plant with a culture product in which the transformant is cultured; and
(e) pulverizing the plant to obtain an influenza virus-derived recombinant hemagglutinin (HA) protein forming a trimer.

In the method for producing an influenza virus-derived recombinant HA protein forming a trimer according to the present invention, (a) and (b) are the same as described above, and thus, the description thereof will be omitted.

In the method for producing an influenza virus-derived recombinant HA protein derived forming a trimer according to the present invention, in step (c), any method known in the art may be appropriately selected and used to produce an influenza virus-derived recombinant HA protein forming a trimer according to the present invention.

In the method for producing an influenza virus-derived recombinant HA protein forming a trimer according to the present invention, step (d) may be carried out, for example, by infiltrating the culture product in which the transformant was cultured into the plant using a chemical cell method, vacuum or syringe infiltration method, and preferably, it may be infiltrated by a syringe infiltration method, but is not limited thereto.

In the method for producing an influenza virus-derived recombinant HA protein forming a trimer according to the present invention, the plant of step (d) may be selected from food crops including rice, wheat, barley, corn, soybean, potato, wheat, red bean, oat and sorghum; vegetable crops including *Arabidopsis thaliana,* Chinese cabbage, radish, red pepper, strawberry, tomato, watermelon, cucumber, cabbage, Korean melon, pumpkin, green onion, onion and carrot; special crops including ginseng, tobacco, cotton, sesame, sugar cane, sugar beet, perilla, peanut and rapeseed; fruit trees including apple tree, pear tree, date tree, peach, grape, tangerine, persimmon, plum, apricot and banana; and flowers including rose, carnation, chrysanthemum, lily and tulip.

A fourth aspect of the present invention relates to an influenza virus-derived recombinant HA protein forming a trimer produced by the above-described production method and a vaccine composition with increased immunogenicity, for preventing, ameliorating or treating influenza virus-infected disease including the same.

In the vaccine composition of the present invention, the influenza virus-derived recombinant HA protein forming the trimer may be coated on the surface of bacteria including peptidoglycan in the cell wall or chitosan. The bacteria including peptidoglycan in the cell wall may be bacteria that are generally recognized as safe (GRAS), and for example, there are *Lactococcus, Lactobacillus, Streptococcus* and the like, but the present invention is not limited thereto.

In a specific exemplary embodiment of the present invention, one or two or more different recombinant HA proteins were coated on the surface of *Lactococcus lactis* in various ways to effectively deliver antigens.

To this end, the recombinant HA protein derived from two or more different types of influenza virus, which forms the trimer of the present invention, may be coated on the surface of bacteria including peptidoglycan in the cell wall or chitosan by any one method of (i) to (iii) below:
i) coating the surface of bacteria including peptidoglycan in the cell wall or chitosan, after mixing two or more different types of influenza virus-derived recombinant HA proteins forming a trimer;
ii) coating the surface of bacteria including peptidoglycan in the cell wall or chitosan with each of two or more different types of influenza virus-derived recombinant HA proteins forming a trimer, followed by mixing; or
iii) coating the surface of bacteria including peptidoglycan in the cell wall or chitosan with two or more different types of influenza virus-derived recombinant.

For example, the vaccine composition of the present invention may be prepared as a single vaccine composition by separately coating *Lactococcus* with two or more different types of influenza virus-derived recombinant HA proteins that form a trimer, and then mixing *Lactococcus* separately coated with each different HA recombination protein at the same ratio (*e.g.,* when mixing *Lactococcus* coated with two different types of HA recombinant proteins, it may be mixed at 1:1), or mixing at any suitable ratio. Alternatively, a single vaccine composition may be prepared by mixing two or more different types of influenza virus-derived recombinant HA proteins at the same ratio or at any suitable ratio, and then coating the surface of *Lactococcus.* Through this, a vaccine composition for delivering multiple antigens may be prepared by additionally mixing *Lactococcus* dead cells coated with two or more different types of influenza virus-derived recombinant HA proteins to other *Lactococcus* dead cells coated with two or more different types of influenza virus-derived recombinant HA proteins.

Therefore, the recombinant HA protein derived from two or more different types influenza viruses included in the vaccine composition according to the present invention may be derived from one or two or more selected from the group consisting of conventionally known conventional influenza virus type A, type B, type C, *Isavirus* and *Thogotovirus.* For example, the influenza A virus may be any one or two or more selected from the group consisting of H5N6, H7N9 and H9N2, but is not limited thereto.

The vaccine composition of the present invention may further include a cholera toxin B subunit. Through this, it is possible to more effectively induce an immune response by increasing the immunogenicity of the recombinant HA protein of the present invention.

The vaccine composition of the present invention may be an injection form, but is not limited thereto.

In the vaccine composition of the present invention, influenza virus-infected disease includes acute respiratory disease caused by influenza virus type A, type B or type C infection or clinical symptoms and complications thereof. Clinical symptoms due to acute respiratory disease caused by influenza virus infection include, for example, respiratory symptoms such as high fever (about 38 to 40°C), dry cough and sore throat, and systemic symptoms such as headache, muscle pain, fatigue, weakness and loss of appetite. The most common complications are secondary respiratory diseases, such as upper respiratory tract infections such as sinusitis and otitis media, as well as neurological complications such as encephalitis, myelitis, Guillain-Barré syndrome, transverse myelitis, myocarditis, myositis, pneumothorax and the like.

As used here, the terms "prevention", "amelioration" and/or "treatment" refer to all actions that inhibit or delays the onset of a disease or condition, all actions that ameliorate or beneficially change the disease or condition state, and all actions that delay, stop or reverse the progression of the disease or condition.

The vaccine composition of the present invention may be composed of an antigen, a pharmaceutically acceptable carrier, an appropriate adjuvant and other conventional substances, and is administered in an immunologically effective amount. As used herein, the term "immunologically effective amount" refers to an amount sufficient to induce an immune response, but not to cause side effects or serious or excessive immune response, and the exact dosage concentration depends on the particular immunogen and may be determined by one of ordinary skill in the art using known methods to test the development of an immune response. In addition, it may change depending on the dosage form and route, the age, health and weight of the recipient, the nature and severity of symptoms, the type of current treatment and the number of treatments.

Carriers are known in the art and may include stabilizers, diluents and buffers. Suitable stabilizers include carbohydrates such as sorbitol, lactose, mannitol, starch, sugar, dextran and glucose; proteins such as albumin or casein. Suitable diluents include salts, Hanks balanced salts, Ringer's solution and the like. Suitable buffers include alkali metal phosphates, alkali metal carbonates, alkaline earth metal carbonates and the like. In addition, the vaccine composition of the present invention may further include one or more selected from the group consisting of a solvent, an adjuvant and an excipient. The solvent includes physiological saline or distilled water, the immune enhancer includes Freund's incomplete or complete adjuvant, aluminum hydroxide gel and vegetable and mineral oils, and the excipient includes aluminum phosphate, aluminum hydroxide or aluminum potassium sulfate, but is not limited thereto, and it may further include a substance used in the preparation of vaccines well known to those skilled in the art.

The vaccine composition of the present invention is administered through a known route of administration. Such methods may include, but are not limited to, oral, transdermal, intramuscular, peritoneal, intravenous, subcutaneous and nasal routes, and it may be administered by any device capable of transporting the active substance to a target cell.

The vaccine composition of the present invention is capable of inducing a humoral or optionally cell-mediated immune response and/or a combination of the two immune responses.

A fifth aspect of the present invention relates to a method for preventing, ameliorating or treating influenza virus-infected disease, including administering the various types of the above-described vaccine composition to a subject in need thereof.

In the method for preventing, ameliorating or treating influenza virus-infected disease according to the present invention, the term "subject" refers to any animal, including humans, already infected with or capable of being infected with influenza virus. Examples include, but are not limited to, humans, dogs, cats, pigs, horses, chickens, ducks, geese, turkeys, seals and the like. By administering the vaccine composition of the present invention to a subject in need thereof, it is possible to effectively prevent or treat acute respiratory disease caused by influenza virus type A, type B or type C infection or clinical symptoms and complications thereof. For example, the vaccine composition of the present invention may treat humans infected with influenza viruses of various influenza virus subtypes or variants. In addition, the composition of the present invention may treat chickens or pigs infected with avian influenza of various influenza virus subtypes or variants. The composition of the present invention may be administered in combination with a conventional therapeutic agent for influenza virus infection and/or cholera toxin B subunit.

The term "administered in combination" means that the vaccine composition of the present invention is administered to a subject in need thereof together with an existing therapeutic agent for influenza virus-infected disease and/or cholera toxin B subunit. Administering each component together means that each component may be administered simultaneously, separately or sequentially to obtain a desired effect.

In the method for preventing, ameliorating or treating influenza virus-infected disease according to the present invention, the vaccine composition may be administered orally, parenterally, by inhalation spray, topically, rectally, nasally, bucally, vaginally or an implanted reservoir. As used herein, the term "parenteral" includes, but is not limited to, subcutaneous, intravenous, intramuscular, intraarticular, intrasynovial, intrasternal, intrathecal, intrahepatic, intralesional and intracranial injections. In particular, the composition may be administered orally, intraperitoneally or intravenously.

In the method for preventing, ameliorating or treating influenza virus-infected disease according to the present invention, the administration of the vaccine composition is preferably performed twice or more. For example, after the initial vaccination, booster injections may be performed about 1 to 4 times at intervals of 1 to 10 weeks, but it may be carried out by those skilled in the art by appropriately modifying the same according to the type of animal.

A sixth aspect of the present invention relates to the use of an influenza virus-derived recombinant HA protein forming a trimer according to the present invention in the preparation of a medicament for preventing, ameliorating or treating influenza virus-infected disease.

Hereinafter, preferred examples are presented to help the understanding of the present invention. However, the following examples are only provided for easier understanding of the present invention, and the contents of the present invention are not limited by the following examples.

### [Modes of the Invention]

### [Example 1]

### Design of hemagglutinin (HA) recombinant genes derived from influenza virus surface proteins forming trimer

In order to induce the expression of the HA of H5N6 or H9N2 in a soluble form in the ER lumen, amino acid positions 17 to 531 of the HA of H5N6 (GenBank: AJD09950.1) and amino acid positions 19 to 524 of the HA of H9N2 (GenBank: AFM47147.1) without any transmembrane domain and ER targeting leader sequence were secured. The ER targeting signal obtained from BiP, which is an *Arabidopsis* protein, was fused to the 5'-terminus of the HA to enable ER targeting. In addition, the first repeat sequence of the three repeat sequences of LysM, which is a *Lactococcus* binding domain, from AcmA of *Lactococcus lactis* was fused to the C-terminus of HA using a linker. Then, mHA was constructed by sequentially fusing an Hisx6 tag and HDEL, which is an ER retention motif, to the C-terminus of LysM for HA purification and high accumulation in ER (FIG. 1a). In order to induce trimer formation of the thus-made HA recombinant protein, tHA was additionally constructed by adding a motif inducing the formation of a homotrimer between HA and LysM from a protein called mouse mCoronin1 (FIG. 1b). For expression, macT was used, and the end of Rd29b was used as a transcription terminator. They were confirmed to show high transcription efficiency. The nucleotide sequences used in the experiment are shown in Table 1 below.

**[Table 1]**

| Name | Sequence (5'→3') | SEQ ID NO: |
|---|---|---|
| HA of H5N6 Nucleotide sequence | gatcagatttgtattggatatcatgcgaacaactctacagagcaggttgatacaataatggaaaaga atgtgaccgttacacatgctcaagacatattggagaagacgcacaacgggaggctttgtgatctca atggagttaaaccacttatacttaaggactgcagtgttgccggttggcttctgggaaacccaatgtgc gatgagtttattcgtgttcccgaatggagctacatcgtcgagagagctaacccagcaaatgatctgt gttatccagggaatctcaacgattacgaggaacttaagcatctactttcgagaatcaaccatttcgaa aagacacttattattcctaagtctagttggcctaatcacgaaacctcccttggtgtaagtgcagcatgc ccttatcaaggtatgccgtcttttttccgaaacgtggtttggttgactaagaagaatgatgcttacccta ctatcaagatgagctataataacaccaacagggaggaccttcttattttgtggggtattcaccattcta acaatgccgctgaacagactaatttgtacaagaaccctactacttatgtttcagtaggcacgtcaactt tgaatcagagattggtcccaaaaatcgctacccgtagtcaagttaatggtcagagagggaggatgg acttcttttggactattcttaagccaaatgatgctatacattttgagagcaatggaaactttatcgctccc gagtacgcctataagatagtaaagaagggcgattccactatcatgaagtctgaaatggaatatggtc attgcaatacgaagtgtcaaaccccgattggtgcaattaactcatctatgcctttccacaacattcacc cactaactatcggagagtgtcctaaatacgtcaaatcaaataaattggtgcttgctacaggtctgcgc aactcccctttaagggagcgaagaagaaagaggggtttgtttggagctattgcaggcttcattgag ggtggatggcaaggtatggtggatggctggtacggatatcatcattcgaatgaacaggggtctggt tatgcagcggatcgggagagtacacaaaaggccatagatggagttaccaacaaggtaaactctatt attgataaaatgaacacacagtttgaggctgtggggagggagttcaacaaccttgaaaggcgtatc gaaaacctcaacaaaaagatggaagacggctttctggacgtttggacttacaacgcagaattgctc gttcttatggaaaacgaacgtactttggatttccatgattctaacgtcaagaatctctacgataaagtg aggctgcaacttagggacaatgcaaaagaactaggtaacggttgctttgaattttatcacaagtgtg ataatgagtgtatggagagtgtaagaaacgggacttatgactaccctcagtatagtgaggaagcta gactcaagcgcgaggagatttccggagttaagcttgaatcaattggaacataccagatt | 1 |
| HA of H5N6 Amino acid sequence | dqicigyhannsteqvdtimeknvtvthaqdilekthngrlcdlngvkplilkdcsvagwllgn pmcdefirvpewsyiveranpandlcypgnlndyeelkhllsrinhfektliipksswpnhetsl gvsaacpyqgmpsffrnvvwltkkndayptikmsynntnredllilwgihhsnnaaeqtnly knpttyvsvgtstlnqrlvpkiatrsqvngqrgrmdffwtilkpndaihfesngnfiapeyayki vkkgdstimksemeyghcntkcqtpigainssmpfhnihpltigecpkyvksnklvlatglrn splrerrrkrglfgaiagfieggwqgmvdgwygyhhsneqgsgyaadrestqkaidgvtnkv nsiidkmntqfeavgrefnnlerrienlnkkmedgfldvwtynaellvlmenertldfhdsnvk nlydkvrlqlrdnakelgngcfefyhkcdnecmesvrngtydypqyseearlkreeisgvkles igtyqi | 2 |
| mCor1 Nucleotide sequence | gtgtctaggcttgaggaagatgttagaaatctcaacgcaattgtccagaaacttcaggaaaggttgg ataggctggaggaaactgttcaagctaag | 3 |
| mCor1 Amino acid sequence | vsrleedvrnlnaivqklqerldrleetvqak | 4 |
| Linker 1 Nucleotide sequence | gaggcagccgc taaggaagct gcagcgaaa | 5 |
| Linker 1 Amino acid sequence | sggsgg | 6 |
| Linker 2 Nucleotide sequence | tcaggaggatcaggagga | 7 |
| Linker 2 Amino acid sequence | sggsgg | 8 |
| BiP Nucleotide sequence | atggctcgct cgtttggagc taacagtacc gttgtgttgg cgatcatctt cttcggtgag[?] tgattttccg atcttcttct ccgatttaga tctcctctac attgttgctt aatctcagaa ccttttttcg ttgttcctgg atctgaatgt gtttgtttgc aatttcacga tcttaaaagg ttagatctcg attggtattg acgattggaa tctttacgat ttcaggatgt ttatttgcgt tgtcctctgc aatagaagag gctacgaagt ta | 9 |
| HDEL Nucleotide sequence | cacgatgagctc | 10 |
| HDEL Amino acid sequence | His-Asp-Glu-Leu | 11 |
| M17 Nucleotide sequence | ggcgtgtgtgtgtgttaaaga | 12 |
| LysM Nucleotide sequence | ggtaatactaactctgggggttcaacgaccaccattacaaacaacaacagtggaacaaattcatctt caaccacctacaccgtgaagagtggcgatacgttgtggggaatcagtcaacgttatggtattagcg ttgctcagatccagtctgcaaataaccttaagtctactataatttatattgggcaaaagctagttctgac tggctcggctagtagcaccaattccggaggtagcaataactcagcttctactacccctacaacctct gtaactccagctaagcctacatcacagactaca | 13 |
| LysM Amino acid sequence | gntnsggstttitnnnsgtnsssttytvksgdtlwgisqrygisvaqiqsannlkstiiyigqklvlt gsasstnsggsnnsasttpttsvtpakptsqtt | 14 |
| MacT Nucleotide sequence | agagatctcctttgccccagagatcacaatggacgacttcctctatctctacgatctagtcaggaagt tcgacggagaaggtgacgataccatgttcaccactgataatgagaagattagccttttcaatttcaga aagaatgctaacccacagatggttagagaggcttacgcagcaggtctcatcaagacgatctaccc gagcaataatctccaggagatcaaataccttcccaagaaggttaaagatgcagtcaaaagattcag gactaactgcatcaagaacacagagaaagatatatttctcaagatcagaagtactattccagtatgg acgattcaaggcttgcttcacaaaccaaggcaagtaatagagattggagtctctaaaaaggtagttc ccactgaatcaaaggccatggagtcaaagattcaaatagaggacctaacagaactcgccgtaaag actggcgaacagttcatacagagtctcttacgactcaatgacaagaagaaaatcttcgtcaacatgg tggagcacgacacgcttgtctactccaaaaatatcaaagatacagtctcagaagaccaaagggca attgagacttttcaacaaagggtaatatccggaaacctcctcggattccattgcccagctatctgtca ctttattgtgaagatagtggaaaaggaaggtggctcctacaaatgccatcattgcgataaaggaaag gccatcgttgaagatgcctctgccgacagtggtcccaaagatggacccccacccacgaggagca tcgtggaaaaagaagacgttccaaccacgtcttcaaagcaagtggattgatgtgacgcaagacgt gacgtaagtatctgagctagtttttatttttctactaatttggtcgtttatttcggcgtgtaggacatggca accgggcctgaatttcgcgggtattctgtttctattccaactttttcttgatccgcagccattaacgactt ttgaatagatacgctgacacgccaagcctcgctagtcaaaagtgtaccaaacaacgctttacagca agaacggaatgcgcgtgacgctcgcggtgacgccatttcgccttttcagaaatggataaatagcctt gcttcctattatatcttcccaaattaccaatacattacactagcatctgaatttcataaccaatctcgata caccaaatcgt | 15 |
| RD29B terminator Nucleotide sequence | aattttactcaaaatgttttggttgctatggtagggactatggggttttcggattccggtggaagtgagt ggggaggcagtggcggaggtaagggagttcaagattctggaactgaagatttggggttttgctttt gaatgtttgcgtttttgtatgatgcctctgtttgtgaactttgatgtattttatctttgtgtgaaaaagagatt gggttaataaaatatttgcttttttggataagaaactcttttagcggcccattaataaaggttacaaatgc aaaatcatgttagcgtcagatatttaattattcgaagatgattgtgatagatttaaaattatcctagtcaa aaagaaagagtaggttgagcagaaacagtgacatctgttgtttgtaccatacaaattagtttagattat tggttaacatgttaaatggctatgcatgtgacatttagaccttatcggaattaatttgtagaattattaatt aagatgttgattagttcaaacaaaaat | 16 |
| HA of H9N2 Nucleotide sequence | gataaaatttgcattggctaccagtcaacaaactccacagaaactgttgatacactagtagaaaaca atgtccctgtgacacataccaaagaattgctccacacagagcacaatggaatgttatgtgcaacaaa cttgggacaccctcttatcctagacacctgcaccattgaagggttggtgtacggcaatccttcctgtg atttgctactgggagggaaagagtggtcttacattgtcgaaagatcatcagctgttaatgggatgtgc taccctggaagggtagagaatctggaagaactcaggtcctttttcagttctgctcgctcctacaaaag actcctactttttccagaccgtacttggaatgtgactttcaatgggacaagcaaagcatgctcaggct cattctacagaagtatgagatggctgacacacaagaacaattcttaccctattcaagacgcccaatat accaacgactggggaaagaatattctcttcatgtggggcatacaccatccacctactgatactgagc aaatgaatctatacaaaaaagctgatacaacaacaagtataacaacggaagatatcaatcgaacttt caaaccagggatagggccaaggcctcttgtcaatggtcaacaaggaagaattgattattattggtca gtactaaagccaggccagacattgcgaataagatccaatggaaatctaattgccccatggtatgga cacattctttcaggagaaagccatggaagaatcctgaagaccgatttgaatagtggcaactgcataa tacaatgccaaactgagaaaggtggtttgaacacgaccttgccattccaaaatgtcagcaaatatgc atttgggaactgtcccaaatatgttggagtgaagagtctcaaactggcagttggtctaaggaatgtg cctgctacatcaggtagagggcttttcggtgccatagctggattcatagaaggaggttggccagga ctagttgcaggctggtacgggtttcagcactcaaatgatcaaggggttggaatagccgcagacaaa gaatcaactcaagaagcagttgataaaataacatccaaagtaaataatataatcgacaaaatgaaca agcagtatgaaatcattgatcatgagttcagtgagattgaagccagactcaatatgatcaacaataa gattgatgaccaaatacaggacatctgggcgtacaatgcagaattactagtactgcttgaaaaccag aaaacactcgatgatcatgatgcaaatgtgaacaatctgtataataaggtgaagagagcattgggtt caaatgcaatagaggatgggaagggatgcttcgagttgtatcacaaatgtgatgatcaatgcatgg aaacaattagaaacgggacttatgacaggctaaagtataaagaagaatcaaaactagaaaggcag aaaatagaaggggtaaaactggagtctgaagaaacatacaagatt | 17 |
| HA of H9N2 Amino acid sequence | dkicigyqstnstetvdtlvennvpvthtkellhtehngmlcatnlghplildtctieglvygnpsc dlllggkewsyiverssavngmcypgrvenleelrsffssarsykrlllfpdrtwnvtfngtskac | 18 |
| | sgsfyrsmrwlthknnsypiqdaqytndwgknilfmwgihhpptdteqmnlykkadtttsitt edinrtfkpgigprplvngqqgridyywsvlkpgqtlrirsngnliapwyghilsgeshgrilkt dlnsgnciiqcqtekgglnttlpfqnvskyafgncpkyvgvkslklavglrnvpatsgrglfgai agfieggwpglvagwygfqhsndqgvgiaadkestqeavdkitskvnniidkmnkqyeiid hefseiearlnminnkiddqiqdiwaynaellvllenqktlddhdanvnnlynkvkralgsnai edgkgcfelyhkcddqcmetirngtydrlkykeesklerqkiegvkleseetyki | |

### [Example 2]

### Expression and identification of hemagglutinin (HA) recombinant genes from influenza virus surface proteins forming trimer

The expressions of mHA and tHA were induced through transient expression in the plant leaves of 4 to 5-week-old tobacco plant *Nicotiana benthamiana* by using vacuum infiltration. Infiltrated leaves were harvested on day 3, 5 and 7 (dpi) after infiltration, respectively, thoroughly ground in liquid nitrogen and dissolved in 3 volumes of buffer. Total soluble proteins from the infiltrated leaf extract was developed by SDS-PAGE and then subjected to Western blot analysis. As confirmed in FIG. 2a, the HA recombinant protein showed clear bands at approximately 85 kDa (mH5N6) and 90 kDa (tH5N6), which are positions corresponding to the sizes predicted by the anti-His antibody. In addition, as confirmed in FIG. 2b, the same was observed when the band was confirmed by staining the same membrane with Coomassie Brilliant Blue (CBB). Judging from the band intensity, the expression levels of these recombinant proteins were estimated to be on the order of 100 µg/g fresh weight in infiltrated leaves.

### [Example 3]

### Confirmation of formation of trimeric structure of hemagglutinin (HA) recombinant genes derived from influenza virus surface proteins forming trimer

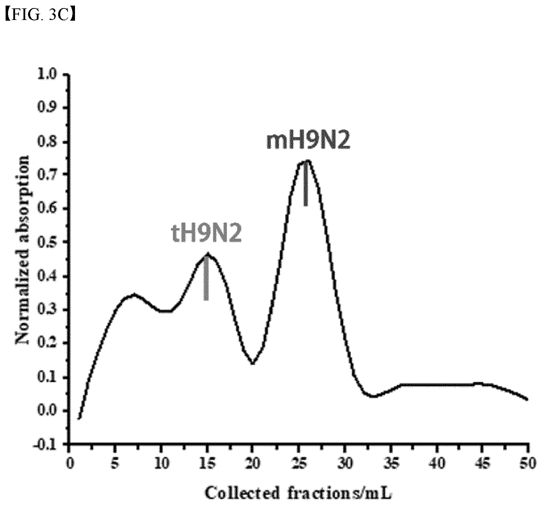

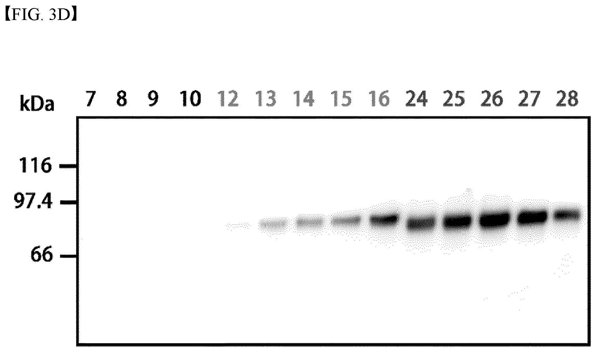

The HA protein on the surface of the virus exists as a homotrimer, exhibiting high stability and immunogenicity. On the other hand, the recombinant HA tends to be expressed as aggregates or monomers, depending on the expression system. In order to mimic the trimer of HA as present on the virus surface, mouse Coronin 1-1A (mCor1, Genbank: EDL17419.1, 32 amino acids), which forms a coiled coil structure and a homotrimer, was added to the C-terminus of HA as shown in FIG. 1b. These genes were introduced into *Nicotiana benthamiana* to induce expression, and total soluble proteins from each 5 dpi leaf tissue were secured and purified by Ni²⁺-NTA affinity column chromatography. Separated and purified mHA and tHA were quantified based on the BSA standard curve. In order to confirm whether HA formed a trimer by mCor1, PBS was added to about 10 µg of mHA and 10 µg of tHA and mixed such that the total volume was 1 mL, and it was analyzed by size exclusion chromatography (SEC). As confirmed in FIGS. 3a and 3c, the mHA and tHA mixture showed two peaks in the 280 nm absorption spectrum. Fractions including these two peaks were analyzed by Western blotting using an anti-His antibody, and the results are shown in FIGS. 3b and 3d, respectively. As shown in the SEC analysis results of FIGS. 3a and 3c and the Western blotting results of FIGS. 3b and 3d, tHAwith mCor1 (*i.e.,* tH5N6 and tH9N2) was eluted before mHA without mCor1 (*i.e*., mH5N6 and mH9N2). Through this, it was confirmed that mCor1 induces the trimer formation of HA.

### [Example 4]

### Identification of the peptidoglycan binding ability of hemagglutinin (HA) recombinant genes derived from influenza virus surface proteins forming trimer

AcmA, which is a major autolysin of MG1363, a phage of *Lactococcus lactis,* has a domain called LysM at the C-terminus in triplicate, and the triplicate part of LysM has the ability to bind to peptidoglycan, which a cell wall component of *Lactococcus.* Three repeats are required for optimal activity on peptidoglycan. However, since the length of the domain is too long when all three repeat sections are included, only one LysM was fused to the C-terminus of GFP and confirmed. As a result, as confirmed in FIGS. 4a and 4b, GFP-LysM did not bind well to *Lactococcus.* Herein, by adding the trimer formation motif of mouse Coronin 1A (mCor-1A) used for the trimer formation of HA, GFP-mCOr1-LysM was constructed to induce the formation of a trimer like the trimer in HA, and afterwards, it was confirmed whether it bound to *Lactococcus.* As a result, as shown in FIGS. 4a and 4b, a significantly increased GFP signal was confirmed in *Lactococcus* compared to GFP-LysM. Through this, since the trimer formation motif of mCor1A induced the trimer formation of GFP, and the GFP trimer thus formed had three LysM motifs, it could be interpreted that GFP binds well to *Lactococcus.* These results are considered to suggest that tHA will bind well to *Lactococcus.*

### [Example 5]

### Identification of the maximum binding amount of hemagglutinin (HA) recombinant genes derived from influenza virus surface proteins forming a trimer

Both mHA and tHA, which are recombinant proteins of HA, were highly expressed in *Nicotiana benthamiana.* Infiltrated leaves were thoroughly ground in liquid nitrogen and dissolved in 10-volume PBS buffer containing 0.5% Triton X-100, 1 mM EDTA and 25% glycerol. After obtaining the total soluble protein from the leaves, and incubating with *L. lactis* pretreated with TCA at 37°C for 1 hour in an amount of the total soluble protein corresponding to 200 mg to 2 g of the leaves, it was washed 3 times with a PBS buffer. *Lactococcus* was precipitated from each sample, placed in an SDS buffer, heated, and then developed by SDS-PAGE with various amounts of BSA, and then stained with Coomassie Brilliant Blue to confirm HA. As a result, as confirmed in FIG. 5, tHA with the trimeric motif mCor1 had an increased amount of binding to *Lactococcus lactis* as the amount of HA increased, whereas mHA hardly bound to Lactococcus. The maximum binding amount of the trimer tHA was estimated to be about 1.7 µg/1 ml *L. lactis* (OD = 1) (FIG. 5).

### [Example 6]

### Preparation of Lactococcus dead cells and method of coating the hemagglutinin (HA) recombinant protein derived from influenza virus surface protein forming a trimer on the dead cells

After culturing *Lactococcus* (Korea Culture Center of Microorganisms; KCCM No. 43146) at OD₆₀₀ to 1.0, the culture solution was recovered by pelleting the cells through centrifugation, and it was resuspended in an equal volume of 10% trichloroacetic acid (TCA) and treated at 100°C for 10 minutes. In order to remove TCA, cells were pelleted through centrifugation, washed three times with PBS, and the pellet was resuspended to prepare *Lactococcus* dead cells. Various amounts of the total soluble protein extract prepared using buffer (PBS pH = 7.5, 1 mM EDTA, Triton X-100, cocktail and 25% glycerol) were added and incubated at 37°C for 1 hour. *Lactococcus* dead cells were pelleted by centrifugation at 12,000 rpm for 5 minutes, and these were washed three times with a PBS buffer for protein extract.

### [Example 7]

### Identification of immunogenicity of antigens forming a trimer using mice

Recombinant tH5N6 prepared in Examples 2 and 6, respectively, tH5N6 coated on *Lactococcus* dead cells, tH9N2, and tH9N2 vaccine coated on *Lactococcus* dead cells were administered to 6-week-old female C57BL/6 mice (Orient Bio, Korea). Intraperitoneal injection was performed twice at an interval of two weeks. As a control group, PBS and *Lactococcus* dead cells were administered. A test vaccine was prepared by adding the recombinant vaccine with or without mixing an adjuvant in the composition shown in Table 2 below.

**[Table 2]**

| **Test group (+adjuvant)** | **Composition** |
|---|---|
| PBS | PBS + Freund's complete adjuvant |
| Lactococcus dead cells | 10⁹ Lactococcus cells + 25% glycerol + PBS + Freund's complete adjuvant |
| tH5N6 | tHA 1 µg of H5N6 + Freund's complete adjuvant + PBS |
| tH5N6 coated on Lactococcus dead cells | tHA 1 µg of H5N6 + 10⁹ Lactococcus cells + 25% glycerol + PBS + Freund's complete adjuvant |
| tH9N2 | tHA 1 µg of H9N2 + PBS |
| tH9N2 coated on Lactococcus dead cells | tHA 1 µg of H9N2 + 10⁹ Lactococcus cells + 25% glycerol + PBS + Freund's complete adjuvant + PBS |

| **Test group (-adjuvant)** | **Composition** |
|---|---|
| PBS | PBS |
| Lactococcus dead cells | 10⁹ Lactococcus cells + 25% glycerol + PBS |
| tH5N6 | tHA 1 µg of H5N6 + PBS |
| tH5N6 coated on Lactococcus dead cells | tHA 1 µg of H5N6 + 10⁹ Lactococcus cells +25% glycerol + PBS |
| tH9N2 | tHA 1 µg of H9N2 + PBS |
| tH9N2 coated on Lactococcus dead cells | tHA 1 µg of H9N2 + 10⁹ Lactococcus cells +25% glycerol + PBS |

In order to analyze the humoral immune response induced by the administered test vaccine, the antigen-specific antibody formation was analyzed by the ELISA method by separating it from the serum of the mice at the 4^{th} week, which was the 2^{nd} week after the administration of the secondary vaccine at 0 week before immunization, to determine the antibody titer. Total IgG antibody titer was determined by the following method.

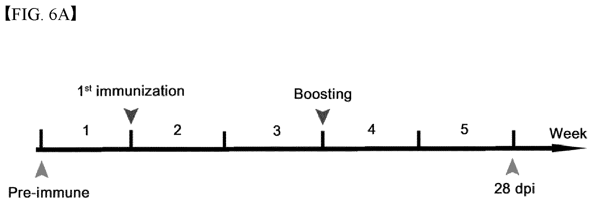

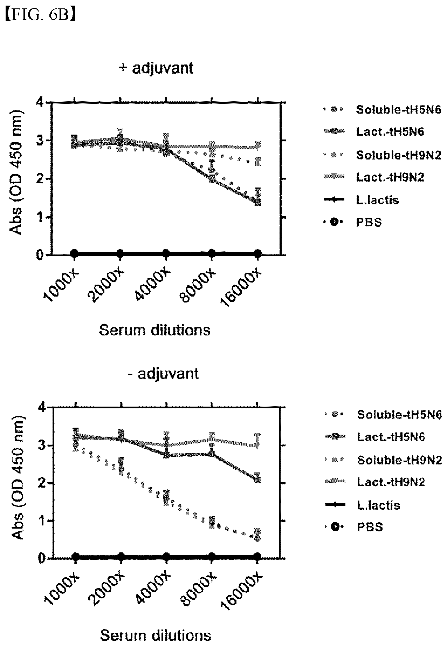

Specifically, after coating the purified recombinant antigen in a 96-well microplate at a concentration of 50 ng/well, 200 µL of a PBST buffer (NaCl 137 mM, KCl 2.7. mM, Na₂HPO₄ 10mM, KH₂PO₄ 1.8mM, Tween 20 1%) containing 3% skim milk was added to prevent non-specific binding, and it was reacted for 2 hours. The microplate was washed, and serum diluted with PBST solution containing 3% skim milk was sequentially added to each well and reacted on a microplate shaker at room temperature for 1 hour. Then, after washing 3 times with 200 µL of a PBST buffer, anti-mouse IgGHRP (horse radish heroxidase, KPL, USA, Bethyl.) was added as a secondary antibody and reacted under the same conditions for 2 hours. After washing the reacted microplate, a color developing reagent TMB (3,3',5,5'-tetramethyl benzidine) and peroxidase substrate (KPL, USA) were added, and the reaction was carried out at room temperature for 10 minutes, and then, the color reaction was stopped using a 0.18M H₂SO₄ stop solution, and OD was measured at 450 nm using an ELISA reader. The antibody titer was defined as the reciprocal of the antibody dilution factor representing an OD value corresponding to twice the OD value of the negative control group. As a result, as confirmed in FIG. 6b, tHA coated on the surface of *Lactococcus* without an adjuvant exhibited the same antibody-inducing effect as that including the adjuvant. Through this, it suggests that the antigen coated on *Lactococcus* can strongly induce immunity without adjuvant, and *Lactococcus* dead cells can be a powerful adjuvant in intraperitoneal and intramuscular administration, which may be a cost-effective application of vaccination.

### [Example 8]

### Comparative analysis of hemagglutinin (HA) recombinant protein derived from influenza virus surface protein coated on the surface of Lactococcus and the degree of inhibition of hemagglutination of the water-soluble trimer

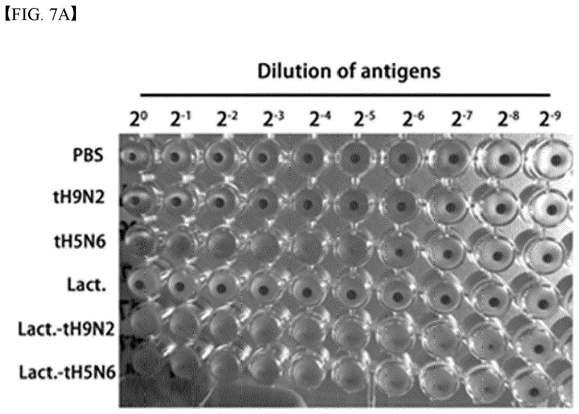

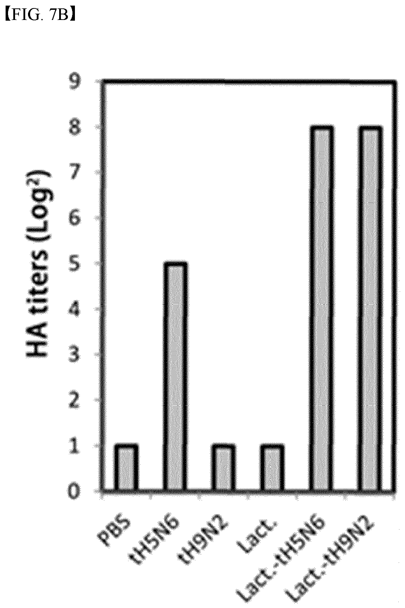

PBS was used as a control group, and by using antigens diluted by a factor of two times of tH9N2 (HA trimer of H9N2), tH5N6 (HA trimer of H5N6), *Lactococcus* dead cells, Lact.-tH9N2 (HA trimer of H9N2 coated on the surface of *Lactococcus*) and Lact.-tH5N6 (HA trimer of H5N6 coated on the surface of *Lactococcus*)*,* hemagglutination inhibition assays were performed to analyze hemagglutination. 25 µL of dilution buffer was added to the U-bottom microplate from the first to the last well. After 25 µL of dilution buffer was added to the first well, the pretreated sample was diluted by two-fold from the first well to the second last well by transferring 25 µL. In this case, 25 µL of the pre-treated sample was added to the last well to confirm the non-specific reaction of the sample. Each antigen diluted to 8 HA Units was added in an amount of 25 µL from the first to the last well, sealed, and incubated at room temperature for 45 minutes. 25 µL of 1% chicken blood cells was added to each well and incubated at room temperature for 1 hour before reading. As confirmed in FIGS. 7a and 7b, even in the hemagglutination inhibition experiment, antigen-coated *Lactococcus,* that is, Lact.-tH9N2 in which the HAtrimer of H9N2 was coated on the surface of *Lactococcus* and Lact. -tH5N6 in which the HA trimer of H5N6 was coated had much higher activities.

### [Example 9]

### Confirmation of antigenicity of trimers coated on the surface of Lactococcus in poultry

Six-week-old chickens (Namduk SPF, Korea) were divided into 5 chickens per group, and PBS, *Lactococcus* dead cells, the HA trimer of soluble H5N6 and the HA trimer of H5N6 coated on the surface of *Lactococcus* were used as antigens, and after administration by intramuscular injection 1 time, the production of antibodies was confirmed.

Similarly, the production of antibodies was confirmed after injection into the muscles of chickens in the same manner as above using the HA of H9N2 as an antigen. The composition of the vaccine used in this example is shown in Table 3 below. In this case, H9N2 virus 1 × 10⁷ EID50 was injected as a control group to confirm the generation of the antibody.

**[Table 3]**

| **Test group** | **Composition** |
|---|---|
| PBS | PBS |
| Lactococcus dead cells | 10⁹ cells of Lactococcus dead cells + 25% glycerol + PBS |
| tH5N6 | tHA 2 µg of H5N6 + PBS |
| tH5N6 coated on Lactococcus dead cells | tHA 2 µg of H5N6 + 10⁹ cells of Lactococcus dead cells + 25% glycerol + PBS |

| **Test group** | **Composition** |
|---|---|
| PBS | PBS |
| Lactococcus dead cells | 10⁹ cells of Lactococcus dead cells + 25% glycerol + PBS |
| tH9N2 | tHA 2 µg of H9N2 + PBS |
| tH9N2 coated on Lactococcus dead cells | tHA 2 µg of H9N2 + 10⁹ cells of Lactococcus dead cells + 25% glycerol + PBS |
| Formalin inactivated H9N2 | Formalin-inactivated H9N2 10⁷ virus particles |

In order to analyze the humoral immune response induced by the administered test vaccine, the antibody titer was determined by analyzing by ELISA for antigen-specific antibody formation after separating the chicken serum at the 2^{nd}, 3^{rd} and 4^{th} weeks of immunization, respectively. Total IgG antibody titer was confirmed by the following method.

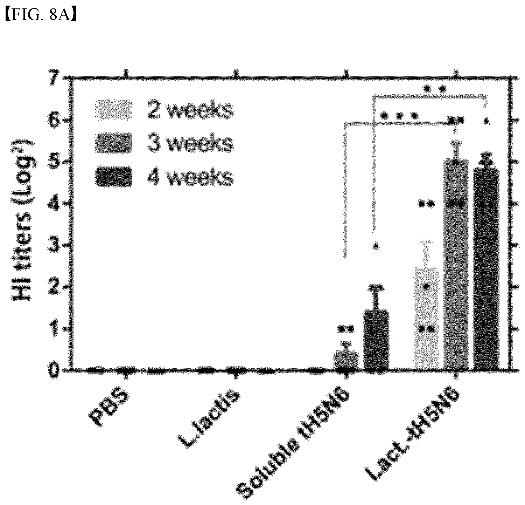

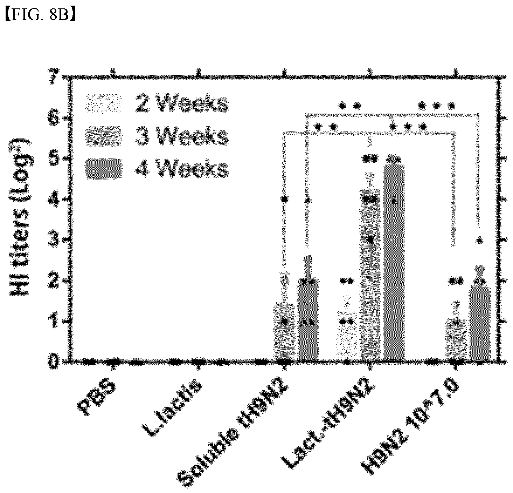

Specifically, the purified recombinant antigen was coated on a 96-well microplate at a concentration of 100 ng/well, and then, 1% bovine serum albumin was added to prevent non-specific binding and reacted for 1 hour. The microplate was washed, serially diluted serum was added to each well, and it was reacted at 37°C for 2 hours, and anti-mouse IgGHRP (horse radish heroxidase, KPL, USA) was added as a secondary antibody, and it was reacted under the same conditions for 1 hour. After washing the reacted microplate, a color developing reagent TMB (3,3',5,5'-tetramethyl benzidine) and peroxidase substrate (KPL, USA) were added, and the reaction was carried out at room temperature for 10 minutes, and the color reaction was stopped using a stop solution, and the OD was measured at 450 nm using an ELISA reader. The antibody titer was defined as the reciprocal of the antibody dilution factor representing an OD value corresponding to twice the OD value of the negative control group. As confirmed in FIG. 8a and 8b, even in chickens, the antigen coated on the surface of *Lactococcus* induced a much stronger immune response than that injected in a soluble form. In particular, it was confirmed that 2 µg of the HA trimer of H9N2 coated on the surface of *Lactococcus* had a much stronger immune effect than that of H9N2 virus 1 × 10⁷ EID50 injection.

### [Example 10]

### Confirmation of immune induction after coating CTB (cholera toxin B subunit), HA trimer of soluble H5N6 and HA trimer of soluble H9N2 on Lactococcus dead cells, respectively

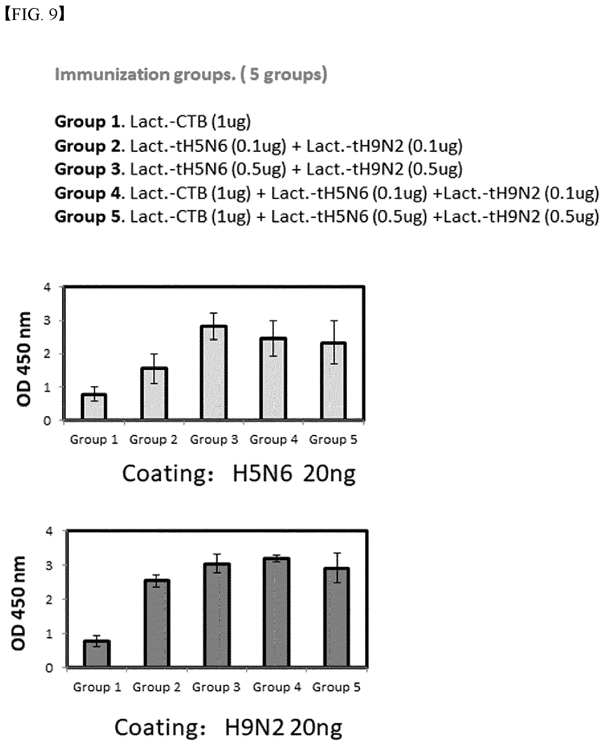

After coating CTB (cholera toxin B subunit), the HA trimer of soluble H5N6 and the HA trimer of soluble H9N2 on *Lactococcus* dead cells, respectively, the immunogenicity was confirmed by antigen intraperitoneal injection of the same into mice (strain name: BALB/c; animal specification: 5 weeks old, female, 20g; Animal purchase: Samtaco, Korea; Number of animals used: 3 animals/group). iLact in which CTB (1 µg) was coated was used as a control group, and iLact-tH5N6 (0.1 µg) + iLact-tH9N2 (0.1 µg), iLact-tH5N6 (0.5 µg) + iLact-tH9N2 (0.5 µg), CTB (1µg) + iLact-tH5N6 (0.1 µg) + iLact-tH9N2 (0.1 µg), or CTB (1µg) + iLact-tH5N6 (0.5 µg) + iLact-tH9N2 (0.5 µg) were prepared as vaccine compositions and intraperitoneally injected twice at an interval of 2 weeks, and afterwards, blood was collected two weeks later and antibodies present in the blood were confirmed by ELISA. In this case, 20 ng of H5N6 and H9N2 antigens were coated on the ELISA plate, respectively. As a result, as shown in FIG. 9, it was confirmed that CTB exhibited the effect of enhancing the immunogenicity of HA when intraperitoneally injected simultaneously with tHA antigens.

### [Example 11]

### Confirmation of immune induction ability after separately coating Lactococcus with HA of H5N6 and HA of H9N2, respectively, and mixing Lactococcus

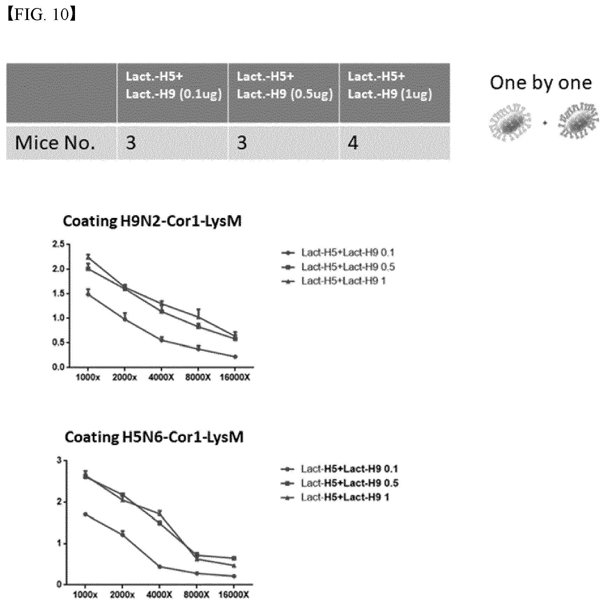

After coating two different types of HA on the surface of *Lactococcus,* the efficacy of immunogenicity on each antigen was confirmed when these were mixed and injected into mice. To this end, iLact-tHA^{H5N6} was prepared by coating 0.1 µg, 0.5 µg or 1.0 µg of tHA of H5N6 on the surface of *Lactococcus,* and similarly, after iLact- tHA^{H9N2} was prepared by coating 0.1 µg, 0.5 µg or 1.0 µg of tHA of H9N2 on the surface of *Lactococcus,* the vaccine composition (iLact-tHA^{H5N6} + iLact-tHA^{H9N2}) was prepared by mixing at 1:1 for each concentration. The prepared composition was intraperitoneally injected into mice at an interval of 2 weeks to induce an immune response, and blood was collected 2 weeks after the second injection to measure the amount of antibody. After 50 ng of the two types of tHA^{H5N6} and tHA^{H9N2} antigens were coated on an ELISA plate, the amount of antibody binding to each antigen was measured in the same manner as described in [Example 7]. As a result, strong immunogenicity was confirmed for both antigens as shown in FIG. 10.

### [Example 12]

### Confirmation of immune induction ability through simultaneous coating of Lactococcus with two types of HA of H5N6 and HA of H9N2

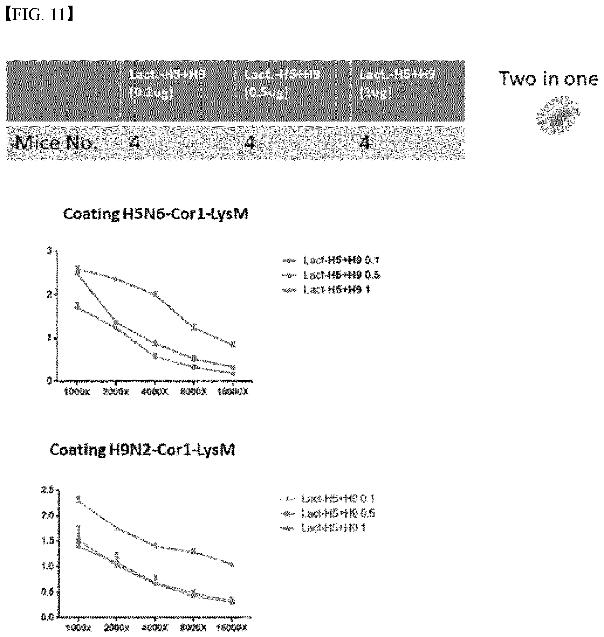

After coating the surface of *Lactococcus* by mixing two types of antigens tHA of H5N6 (tHA^{H5N6}) and tHA of H9N2 (tHA^{H9N2}) having a concentration of 0.1 µg, 0.5 µg or 1.0 µg, respectively, at a ratio of 1:1, [iLact(tHA^{H5N6}) + tHA^{H9N2})] was intraperitoneally injected into mice at an interval of 2 weeks, and blood was collected 2 weeks after the secondary immunization injection to confirm the formation of antibodies through ELISA. In the ELISA, 50 ng of the antigen used was coated, blood was diluted at an appropriate ratio, and the amount of antibody present in the blood was measured in the same manner as described in [Example 7]. As shown in FIG. 11, it was confirmed that the vaccine composition prepared by the method of this example induces a strong immune response to both antigens. Therefore, it was confirmed that *Lactococcus* can simultaneously deliver the two types of antigens (tHA^{H5N6} and tHA^{H9N2}).

## Claims

1. A recombinant vector for producing an influenza virus-derived recombinant hemagglutinin (HA) protein forming a trimer, comprising:
(i) a gene encoding a protein lacking a transmembrane protein portion in influenza virus-derived hemagglutinin (HA); and
(ii) a gene encoding a protein of a trimeric motif region of Coronin 1.

2. The recombinant vector of claim 1, wherein the influenza virus is any one selected from the group consisting of Influenza A viruses H5N6, H7N9 and H9N2.

3. The recombinant vector of claim 1, wherein the protein lacking a transmembrane protein portion in influenza virus-derived HA comprises the amino acid sequence of SEQ ID NO: 2 or the amino acid sequence of SEQ ID NO: 18.

4. The recombinant vector of claim 1, wherein the gene encoding a protein lacking a transmembrane protein portion in influenza virus-derived HA comprises the nucleotide sequence of SEQ ID NO: 1 or the nucleotide sequence of SEQ ID NO: 17.

5. The recombinant vector of claim 1, wherein the protein of a trimeric motif region of Coronin 1 comprises the amino acid sequence of SEQ ID NO: 4.

6. The recombinant vector of claim 1, wherein the gene encoding a protein of a trimeric motif region of Coronin 1 comprises the nucleotide sequence of SEQ ID NO: 3.

7. The recombinant vector of claim 1, further comprising a gene encoding a protein of the LysM domain in the recombinant vector.

8. The recombinant vector of claim 7, wherein the protein of the LysM domain comprises the amino acid sequence of SEQ ID NO: 14.

9. The recombinant vector of claim 7, wherein the gene encoding a protein of the LysM domain comprises the nucleotide sequence of SEQ ID NO: 13.

10. A transformant which is transformed by the recombinant vector according to any one of claims 1 to 9.

11. A method for producing an influenza virus-derived recombinant hemagglutinin (HA) protein forming a trimer in a plant, comprising the steps of:
(a) constructing the recombinant vector according to claim 1;
(b) introducing the recombinant vector into a cell to prepare a transformant;
(c) culturing the transformant;
(d) infiltrating a plant with a culture product in which the transformant is cultured; and
(e) pulverizing the plant to obtain an influenza virus-derived recombinant hemagglutinin (HA) protein forming a trimer.

12. An influenza virus-derived recombinant HA protein forming a trimer, which is produced by the method of claim 11.

13. A vaccine composition with increased immunogenicity, for preventing or treating influenza virus-infected disease, comprising the influenza virus-derived recombinant HA protein forming a trimer according to claim 12.

14. The vaccine composition with increased immunogenicity, for preventing or treating influenza viruses-infected diseases caused by influenza viruses having different genotypes of claim 13, wherein the influenza virus-derived recombinant HA protein forming a trimer comprises two or more different types of influenza virus-derived recombinant HA proteins.

15. The vaccine composition of claim 13 or 14, wherein the influenza virus-derived recombinant HA protein forming a trimer is coated on the surface of bacteria including peptidoglycan in the cell wall or chitosan.

16. The vaccine composition of claim 15, wherein the bacteria including peptidoglycan in the cell wall is bacteria which is generally recognized as safe (GRAS).

17. The vaccine composition of claim 13 or 14, further comprising a cholera toxin B subunit.

18. The vaccine composition of claim 13 or 14, wherein the vaccine composition is an injection form.

19. The vaccine composition with increased immunogenicity, for preventing or treating influenza viruses-infected diseases caused by influenza viruses having different genotypes of claim 14, wherein the two or more different types of influenza virus-derived recombinant HA proteins forming a trimer are coated on the surface of bacteria including peptidoglycan in the cell wall or chitosan by any one method of i) to iii) below:
i) coating the surface of bacteria including peptidoglycan in the cell wall or chitosan, after mixing two or more different types of influenza virus-derived recombinant HA proteins forming a trimer;
ii) coating the surface of bacteria including peptidoglycan in the cell wall or chitosan with each of two or more different types of influenza virus-derived recombinant HA proteins forming a trimer, followed by mixing; or
iii) coating the surface of bacteria including peptidoglycan in the cell wall or chitosan with two or more different types of influenza virus-derived recombinant HA proteins forming a trimer by the two methods of (i) and (ii) above.

20. A method for preventing or treating influenza virus-infected disease, comprising administering the vaccine composition according to any one of claims 13 to 19 to a subject in need thereof.
